# FASCICULE DE BREVET EUROPEEN

(11) **EP 3 232 926 B1**
(45) Date de publication et mention de la délivrance du brevet: **14.08.2019**
(21) Numéro de dépôt: 15822981.5
(22) Date de dépôt: 16.12.2015
(51) Int. Cl.: A61B 5/11, G01V 3/08, G08B 13/26, G08B 21/04

(54) **CAPTEUR CAPACITIF A DEUX DIMENSIONS POUR LOCALISER LA PRESENCE D'UN OBJET ET/OU D'UN INDIVIDU**
ZWEIDIMENSIONALER KAPAZITIVER SENSOR ZUR DETEKTION DER ANWESENHEIT EINES OBJEKTS UND/ODER EINES INDIVIDUUMS
TWO-DIMENSIONAL CAPACITIVE SENSOR FOR LOCATING THE PRESENCE OF AN OBJECT AND/OR OF AN INDIVIDUAL

(30) Priorité: 19.12.2014 FR 1462909
(43) Date de publication de la demande: 25.10.2017
(73) Titulaire: BOSTIK SA, 92700 Colombes (FR)
(72) Inventeur: CASIMIRO, Jessie, 77170 Brie Comte Robert (FR); MABIRE, Philippe, 91080 Courcouronnes (FR); HAFFNER, Julien, 77420 Champs Sur Marne (FR); MARGO, Cédric, 91400 Orsay (FR); OUSSAR, Yacine, 75013 Paris (FR); HOLE, Stéphane, 75013 Paris (FR)
(74) Mandataire: Cabinet Plasseraud
(86) Numéro de dépôt international: PCT/FR2015/053555
(87) Numéro de publication internationale: WO 2016/097611

(56) Documents cités:
- EP-A2- 0 161 895
- WO-A1-2014/053719
- DE-A1-102009 055 121
- DE-U1- 20 211 697
- US-A- 5 010 772
- US-B1- 8 266 971

## Description

### Domaine technique

La présente invention se situe dans le domaine de la détection de présence d'objets et/ou d'individus.

Un des objectifs de la présente invention est d'instrumenter un sol avec un ou plusieurs capteurs aptes à détecter et localiser la présence d'un objet et/ou d'un individu.

L'objet de la présente invention trouve ainsi une application avantageuse pour détecter et localiser la chute d'un individu dans une pièce dont le sol est instrumenté avec un tel agencement de capteurs : la présente invention est donc particulièrement intéressante pour les bâtiments, médicalisés ou non, dédiés aux personnes âgées tels que par exemple les maisons de retraite.

Bien évidemment, d'autres applications avantageuses peuvent être envisagées dans le cadre de la présente invention, notamment :
- dans le domaine de la sécurité (par exemple dans les musées, les maisons individuelles, les locaux publics, etc.), ou encore
- dans le domaine de l'automobile.

### Arrière-plan technologique

Les conditions d'hygiène et de santé s'améliorent dans la plupart des pays ; ceci a pour conséquence directe un accroissement de l'espérance de vie.

Ainsi, en Europe, l'âge moyen de la population augmente régulièrement.

Les projections à l'horizon 2060 révèlent que le pourcentage de personnes ayant plus de 65 ans devrait atteindre plus de 50 % de la population totale, contre à peine 20 % actuellement.

Ce vieillissement général de la population incite les acteurs de la santé à trouver des solutions pour assurer aux personnes âgées leur indépendance le plus longtemps possible, et ce avec un minimum d'assistance.

Parmi les différentes problématiques soulevées par ce vieillissement général, un des enjeux est de mettre en place des systèmes performants pour détecter les chutes.

En effet, tous les ans, on recense de nombreuses chutes impliquant une hospitalisation car la chute n'a pas été détectée à temps : on notera ici que la santé d'une personne âgée peut se dégrader très vite lorsque la prise en charge après la chute est trop tardive.

Il existe dans l'état de la technique plusieurs systèmes de détection de chute.

Parmi ces systèmes, on retrouve :
- ceux qui doivent être portés en permanence tels que par exemple les patchs « antichute », ou encore
- ceux qui sont intrusifs tels que par exemple les systèmes de télésurveillance avec traitement d'images.

Il existe d'autres systèmes de détection évitant les inconvénients ci-dessus.

A cet effet, le document WO 2006/130081 propose une méthode de détection de personne au lever du lit. La méthode proposée dans ce document est particulièrement adaptée aux personnes âgées et/ou aux handicapés.

Plus précisément, des capteurs de pression, introduits dans une mousse de polyuréthane, sont reliés à un système de surveillance qui déclenche une alarme lorsqu'une pression est exercée sur les capteurs.

Néanmoins, cette méthode utilisant des capteurs de pression n'est pas adaptée aux détections de chute. En effet, avec une telle méthode, il est impossible de différencier une personne qui marche d'une personne qui tombe.

Le document WO 2009/050285 propose quant à lui un tapis de sol instrumenté par un système composé de capteurs. Ce système utilise la capacitance en relation avec la déformation d'une couche intermédiaire pour détecter la présence indifféremment d'une personne ou d'un objet.

Néanmoins, les capteurs introduits dans ce tapis ne sont pas adaptés pour détecter spécifiquement les transferts de charges liés à la présence d'une personne ou d'un objet.

Bien qu'intéressantes, les différentes solutions ci-dessus ne permettent pas une détection fine des chutes de personnes. Par ailleurs, l'installation des solutions proposées ci-dessus est très coûteuse, difficile et fastidieuse à mettre en oeuvre.

Alternativement, l'incorporation de capteurs capacitifs dans le sol d'une pièce permet de détecter de manière non-intrusive la présence de personnes à la surface en mesurant la variation d'une grandeur physique.

En effet, une personne au sol est assimilable à une variation locale de la permittivité ou à la présence d'une nouvelle électrode.

Dans ce contexte, les capteurs capacitifs sont des capteurs de choix pour la détection de personnes. En effet, les capteurs capacitifs fonctionnent comme des condensateurs et leur capacité varie lorsqu'un objet ou un individu se rapproche : cette variation de la capacité permet de déterminer la présence ou non d'un objet ou d'un individu à proximité du capteur.

Le document FR 2 956 137 propose ainsi un sol instrumenté avec de tels capteurs capacitifs pour la détection de présence.

Dans ce document, le sol comprend :
- une sous-couche constituée dans un matériau électriquement isolant et posée sur une dalle,
- des capteurs capacitifs posés sur la sous-couche, et
- une chape posée sur la sous-couche, cette chape étant isolante et recouvrant les capteurs.

Selon ce document FR 2 956 137, il est indiqué comme essentiel de disposer d'une sous-couche constituée d'un matériau électriquement isolant pour permettre une détection efficace avec les capteurs capacitifs.

Dans ce document, l'installation d'un sol instrumenté avec une sous-couche électriquement isolante est complexe et onéreuse.

Le document WO 2014/053719 propose une solution pour éviter une telle sous-couche. Ainsi, dans ce document, il est prévu un capteur capacitif comprenant un agencement spécifique d'une pluralité d'électrodes.

Plus particulièrement, dans ce document, le capteur comprend au moins trois électrodes s'étendant longitudinalement dans une seule et même direction.

Selon une première caractéristique avantageuse de ce document, les électrodes du capteur sont polarisées indépendamment les unes des autres pour permettre une détection fine de la chute d'un individu ou d'un objet sur le sol.

Selon une deuxième caractéristique avantageuse de ce document, la géométrie des électrodes (rayon de courbure des électrodes et rapport entre les distances de séparation) permet d'améliorer la précision de la détection.

En tout état de cause, les caractéristiques avantageuses proposées dans ce document WO 2014/053719 ne permettent pas de localiser la position précise de l'individu ou de l'objet une fois celui-ci détecté.

Le document US 5010772 propose un capteur capacitif pour détecter, localiser la présence, et/ou suivre l'activité d'un individu et/ou d'un objet, ledit capteur comportant une première couche comprenant au moins une électrode, dite première électrode, qui s'étend dans une première direction, et une deuxième couche présentant au moins une électrode, dite deuxième électrode, qui s'étend dans une deuxième direction. La première direction est différente de la deuxième direction. Le capteur comprend un module électronique de pilotage configuré pour appliquer de manière indépendante une tension sur chacune des électrodes, et pour mesurer les charges électriques accumulées pour chaque électrode. Le module de pilotage est configuré pour appliquer une tension nulle sur au moins l'une des électrodes.

La Demanderesse soumet qu'aucun des documents de l'état de la technique ne propose une solution permettant de détecter et de localiser avec précision la présence d'un objet et/ou d'un individu sur le sol.

### Objet et résumé de la présente invention

La présente invention vise à améliorer la situation décrite ci-dessus.

Un des objectifs de la présente invention est de remédier aux différents inconvénients mentionnés ci-dessus en proposant un capteur simple à installer permettant de localiser la présence d'un objet et/ou d'un individu.

A cet effet, l'objet de la présente invention concerne selon un premier aspect un capteur capacitif pour détecter, localiser la présence, évaluer la surface au sol et/ou suivre l'activité d'un individu et/ou d'un objet.

La surface d'un individu et/ou d'un objet est ici assimilable à la surface du sol qui est recouverte par l'individu et/ou l'objet sur le sol.

Plus particulièrement, le capteur selon l'invention comporte :
- une première couche comprenant au moins une électrode, dite première électrode, qui s'étend dans une première direction, et
- une deuxième couche présentant au moins une électrode, dite deuxième électrode, qui s'étend dans une deuxième direction.

Selon l'invention, la première direction est différente de la deuxième direction.

En d'autres termes, la première électrode s'étend dans une direction qui n'est pas identique à la deuxième direction ; c'est-à-dire qui n'est pas parallèle ou confondue à la deuxième direction dans laquelle s'étend la deuxième électrode.

Selon l'invention, la première couche est électriquement isolée de la deuxième couche.

On notera ici que, de la même façon, lorsque la couche comporte plus d'une électrode, les électrodes de chaque couche sont avantageusement isolées électriquement les unes des autres.

Le capteur selon la présente invention est donc composé de deux couches, isolées électriquement l'une de l'autre, et comprenant chacune au moins une électrode.

Les électrodes de chaque couche s'étendent dans deux directions de l'espace qui sont différentes ; ceci permet d'avoir un capteur à deux dimensions pour localiser avec précision la position sur le sol instrumenté d'un objet et/ou d'un individu.

Ainsi, grâce à cet agencement de moyens techniques, caractéristique de la présente invention, on dispose d'un capteur :
- qui peut facilement instrumenter un sol : chacune des couches du capteur peut être intégrée directement dans une des parties du sol : chape, ragréage, colle, revêtement de sol ; et
- qui permet non seulement de détecter la présence d'un objet et/ou d'un individu, mais également d'avoir une estimation précise de la position et de la surface de l'objet et/ou de l'individu détecté sur le sol ;
- qui permet d'effectuer le suivi de l'activité d'une personne.

De préférence, les première et deuxième directions sont sensiblement perpendiculaires entre elles.

Cette géométrie des électrodes de la première couche par rapport à la deuxième couche permet d'obtenir la localisation la plus fine.

Avantageusement, la première électrode et/ou la deuxième électrode se présentent sous la forme d'un filament ; c'est-à-dire un mono-filament (l'électrode est formée par un unique fil électrique).

Avantageusement, la première électrode et/ou la deuxième électrode se présentent sous la forme d'une nappe de filaments.

Avantageusement, les première et deuxième couches s'étendent dans des plans sensiblement parallèles entre eux.

Dans un mode de réalisation préféré, les première et deuxième couches sont superposées.

Selon l'invention, il est préférable que chaque électrode des première et deuxième couches soit isolée électriquement par une gaine protectrice.

De préférence, cette gaine protectrice est constituée au moins partiellement dans un matériau polymérique.

Avantageusement, les gaines peuvent être maintenues ensemble par un support.

Ce support permet donc de maintenir en position les gaines entre elles (et donc les électrodes) ; ceci permet avantageusement de conserver une distance de séparation sensiblement constante (par exemple environ 25 centimètres) entre les électrodes de sorte que les électrodes de chaque couche restent sensiblement parallèles entre elles.

Optionnellement, chaque support présente au moins une face auto-adhésive.

Avantageusement, chaque support présente une structure perforée ou aérée, comme dans le cas par exemple d'un tissu ou d'un filet de verre.

Une telle structure participe à la tenue mécanique de la partie du sol dans laquelle le support est intégré.

Optionnellement, chaque support comprend un filet à maille centimétrique.

Dans une variante, le filet à maille est constitué au moins partiellement dans un matériau tel que par exemple un matériau sélectionné parmi les matériaux suivants : fibre de verre, polyester, polypropylène, polyéthylène, ou encore polyamide.

D'autres matériaux assurant une bonne mécanique pourront également être envisagés dans le cadre de la présente invention.

Dans un mode de réalisation, chaque électrode des première et deuxième couches est isolée électriquement par une gaine protectrice, chaque gaine entourant une électrode.

De préférence, pour chaque couche, le filet à maille et les électrodes sont assemblés entre eux par un tissage du type fils de chaîne et fil de trame, lesdits fils de chaîne et ledit fil de trame étant formés respectivement par les gaines et le filet à maille.

Le capteur selon la présente invention comprend un module électronique de pilotage.

Selon l'invention, ce module de pilotage est configuré :
- pour appliquer de manière indépendante une tension sur chacune des électrodes, et
- pour mesurer les charges électriques accumulées pour chaque électrode.

Selon l'invention, le module de pilotage est configuré pour appliquer une tension nulle sur au moins l'une des électrodes.

Avantageusement, la tension appliquée aux électrodes est variable dans le temps, de sorte que la tension équivalente de tous les conducteurs ou diélectriques de grande permittivité et de grande taille peut être considérée comme nulle, si elle ne varie pas à la même fréquence que la tension appliquée aux électrodes.

La fréquence de variation de la tension appliquée aux électrodes est généralement comprise entre 10 Hz et 10 MHz.

Le capteur selon la présente invention comprend en outre un module informatique de traitement qui est configuré pour détecter, localiser la présence, évaluer la surface au sol et/ou suivre l'activité d'un individu et/ou d'un objet en fonction de la distribution spatiale des variations des charges mesurées pour chacune desdites électrodes.

Ainsi, par rapport au document WO 2014/053719 dans lequel on mesure des différences de phases de mesure pour détecter un objet et/ou un individu, la présente invention permet, elle, en plus de localiser et de suivre l'activité d'un objet et/ou d'un individu en analysant l'évolution des charges électriques de chaque électrode dans l'espace.

Lorsqu'on réalise une mesure sur une électrode, on parle d'électrode de mesure.

Le module informatique de traitement est ainsi configuré pour analyser la distribution spatiale des variations des charges mesurées pour chacune desdites électrodes en déterminant :
- une information dépendante du couplage à la terre d'une première électrode de mesure si la tension appliquée sur la première électrode de mesure n'est pas nulle, ladite première électrode de mesure étant comprise dans la première couche,
et, suivant l'information dépendante du couplage à la terre déterminée, en déterminant:
- une information indépendante du couplage à la terre d'une deuxième électrode de mesure si la tension appliquée sur la deuxième électrode de mesure est nulle et que la tension appliquée sur la première électrode de mesure n'est pas nulle, ladite deuxième électrode de mesure étant comprise dans la deuxième couche ; on parlera dans la suite de la description d' « inter-capacité » pour désigner cette information.

Par terre, on entend tous conducteurs ou diélectriques de grande permittivité et de grande taille dont le potentiel n'est pas affecté par les tensions appliquées sur une ou plusieurs des quelconques électrodes du capteur.

Dans un mode de réalisation particulier, le capteur comprend en outre un module d'affichage comprenant un écran de contrôle.

Ce module est apte à coopérer avec le module informatique de traitement pour générer sur l'écran une imagerie représentative de la position spatiale de l'objet et/ou de l'individu en fonction de la distribution spatiale des variations des charges mesurées pour chacune des électrodes.

L'écran de contrôle peut être déporté dans un centre de contrôle avec une communication par réseau informatique des données entre l'écran et le capteur ; un tel écran permet à un opérateur d'intervenir en cas de chute détectée.

Un tel écran de contrôle est optionnel ; on peut prévoir que l'opérateur intervienne simplement suite au déclenchement d'une alarme lorsqu'une chute a été détectée par exemple.

Corrélativement, l'objet de la présente invention concerne selon un deuxième aspect une structure de sol pour localiser un objet et/ou un individu.

Par sol au sens de la présente invention, on entend ici tout système qui présente une structure comprenant notamment une chape (ou couche de chape), une couche de primaire d'accrochage, éventuellement une barrière d'étanchéité à l'eau, une couche de ragréage, une couche de colle, et/ou une couche de revêtement.

Selon la présente invention, la structure de sol comporte au moins un capteur capacitif tel que décrit ci-dessus.

L'instrumentation du sol avec au moins un capteur capacitif tel que décrit ci-dessus permet d'obtenir un système particulièrement simple à installer, un tel sol étant adapté pour localiser la présence d'un objet et/ou d'un individu.

Grâce à la présente invention, une instrumentation du sol peut être envisagée pour des locaux habités qui sont en cours de rénovation ou des bâtiments en cours de construction.

Le capteur capacitif peut être noyé dans une enveloppe protectrice qui peut être polymérique.

De préférence, cette enveloppe protectrice est autoadhésive.

Cette variante est particulièrement intéressante pour la mise en oeuvre de l'invention dans la construction d'un bâtiment ou au moins lors la rénovation des sols. Une telle installation est particulièrement robuste.

Dans un mode de réalisation, la structure de sol présente une couche de revêtement.

Dans ce mode, le capteur capacitif peut être intégré directement dans cette couche de revêtement.

Dans un autre mode alternatif, le capteur peut optionnellement être fixé par collage, directement ou indirectement, sur au moins une portion de la face inférieure de la couche de revêtement.

Par couche de revêtement, on peut comprendre ici par exemple et de manière non limitative une couche telle qu'un parquet (flottant ou non), un carrelage, un revêtement souple comme de la moquette tricotée, touffetée, tissée, floquée, en lés ou en dalles, un revêtement de sol aiguilleté, en lés ou en dalles, un revêtement de sol homogène ou hétérogène à base de polychlorure de vinyle, un revêtement de sol à base de polychlorure de vinyle sur support de jute ou de polyester ou sur support de polyester avec envers en polychlorure de vinyle, un revêtement de sol à base de polychlorure de vinyle sur mousse, un revêtement de sol à base de polychlorure de vinyle avec support à base de liège, un revêtement de sol à base de polychlorure de vinyle expansé, une dalle semi flexible à base de polychlorure de vinyle ou encore une dalle d'aggloméré de liège avec couche d'usure à base de polychlorure de vinyle.

Dans un autre mode de réalisation qui peut être combiné avec l'un des précédents modes, la structure de sol présente une couche de ragréage.

Le capteur peut optionnellement être fixé par collage, directement ou indirectement, sur au moins une portion de la face supérieure de la couche de ragréage.

De préférence, cette couche de ragréage est constituée au moins partiellement de mortier à base de liant organique et/ou minéral.

Il est possible que, dans une variante, la structure de sol présente une couche de ragréage fixée à la couche de revêtement par l'intermédiaire d'une couche de colle. Dans ce cas, le capteur capacitif peut être noyé au moins partiellement dans la couche de colle.

Dans une variante, la structure de sol présente une couche de chape et une couche de ragréage.

Dans cette variante, le capteur est intégré dans la couche de ragréage par coulage de ladite couche de ragréage, ledit capteur étant fixé par collage sur au moins une portion de la face supérieure de la couche de chape.

Une couche de ragréage est ensuite coulée sur le capteur.

Dans cette variante, le capteur est alors intégré dans la couche de ragréage.

Alternativement, il est possible de ne pas avoir de couche de ragréage et d'introduire directement le capteur dans la couche de chape. Ceci implique d'avoir une couche de chape suffisamment lisse pour la suite, notamment pour poser le revêtement.

Ainsi, la structure de sol selon la présente invention propose plusieurs alternatives possibles permettant une instrumentation du sol pour la détection de présence d'objet et/ou d'individu.

Enfin, l'objet de la présente invention concerne selon un troisième aspect un procédé de fabrication d'une structure de sol telle que décrite ci-dessus.

Selon l'invention, le procédé comporte notamment :
- une première étape au cours de laquelle une première couche comprenant au moins une première électrode est positionnée de sorte que la première électrode s'étende dans une première direction déterminée, et
- une deuxième étape au cours de laquelle une deuxième couche comprenant au moins une deuxième électrode est positionnée de sorte que la deuxième électrode s'étende dans une deuxième direction différente de la première direction.

Selon l'invention, les deux couches sont électriquement isolées l'une de l'autre.

De préférence, lors de la deuxième étape, la deuxième couche est positionnée de sorte que la deuxième direction dans laquelle s'étend la deuxième électrode soit sensiblement perpendiculaire à la première direction dans laquelle s'étend la première électrode.

Grâce à ses différentes caractéristiques structurelles et fonctionnelles, l'objet de la présente invention propose donc un capteur, simple à mettre en place dans une pièce et peu coûteux, qui en se déployant dans deux directions permet de réaliser une localisation précise de l'objet et/ou de l'individu se situant dans la pièce. Il est alors possible d'en traiter les informations recueillies et de suivre l'activité de l'objet et/ou de l'individu, et d'établir une imagerie représentative de cette activité.

### Description des figures annexées

D'autres caractéristiques et avantages de la présente invention ressortiront de la description ci-dessous, en référence aux figures 1 à 7 annexées qui en illustrent différents exemples de réalisation dépourvus de tout caractère limitatif et sur lesquelles :
- la figure 1 représente de façon schématique une vue de dessus d'un capteur capacitif selon un exemple de réalisation de la présente invention ;
- la figure 2 représente de façon schématique une vue latérale d'un capteur capacitif selon un exemple de réalisation de la présente invention ;
- les figures 3a à 3c représentent chacune une vue schématique en coupe d'une structure de sol conforme à plusieurs exemples de réalisation de la présente invention ;
- la figure 4 représente une vue schématique du pilotage d'un capteur selon l'état de l'art ;
- la figure 5 représente une vue schématique du pilotage d'un capteur selon un deuxième exemple de réalisation ;
- la figure 6 représente une vue schématique d'une couche d'électrodes d'un capteur assemblées entre elles par tissage avec une fibre de verre ;
- la figure 7 représente une vue schématique d'une couche d'électrodes d'un capteur collées sur un grillage.

### Description détaillée de différents exemples de réalisation avantageux

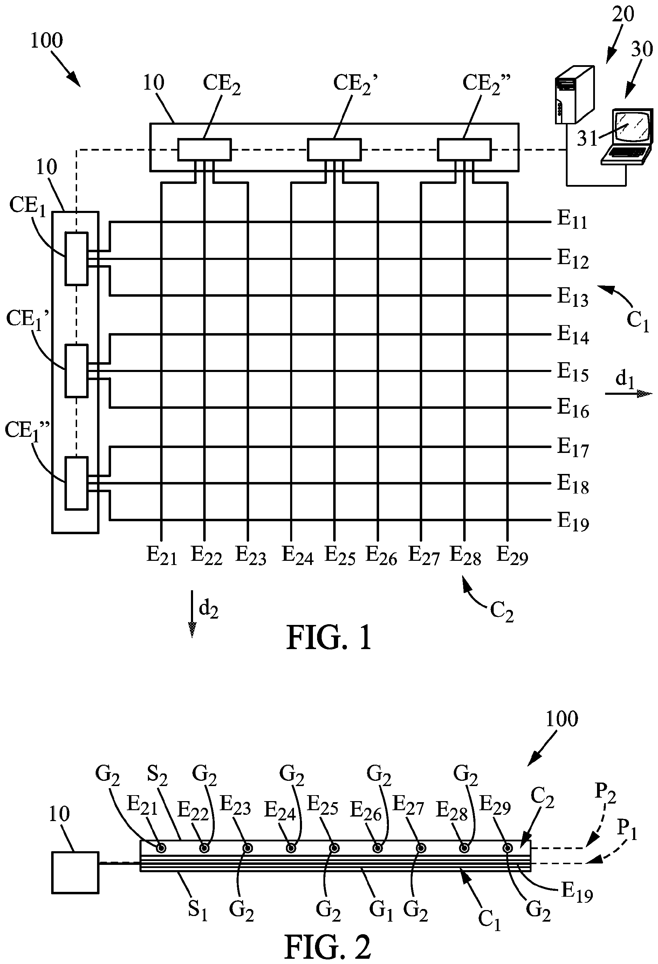

Un capteur capacitif et une structure de sol conformes à différents exemples de réalisation avantageux de la présente invention vont maintenant être décrits en faisant référence conjointement aux figures 1 à 7.

Les exemples décrits ici sont particulièrement adaptés pour une application du type détection et localisation d'une chute d'une personne âgée dans une enceinte médicalisée du type maison de retraite.

Bien évidemment, il s'agit ici d'un exemple d'application qui est purement illustratif ; comme évoqué précédemment, d'autres applications peuvent également être envisagées dans le cadre de la présente invention.

Pour rappel, détecter, localiser spatialement, estimer la surface au sol et suivre l'activité d'un objet et/ou un individu à proximité du sol est un des objectifs de la présente invention.

Concevoir un capteur facile à installer et permettant d'instrumenter un sol est également un des autres objectifs de la présente invention.

Dans l'exemple décrit ici, on prévoit donc la conception d'un capteur capacitif 100 répondant à ces différents problèmes.

Plus particulièrement, dans cet exemple, le capteur 100 est destiné à instrumenter un sol pour localiser la présence et déterminer la surface occupée par un individu et/ou un objet à proximité du sol.

Un capteur capacitif fonctionne comme un condensateur : on sait en effet que la capacité d'un capteur capacitif varie lorsqu'un individu ou un objet se rapproche ou s'éloigne du capteur.

Une application bien connue des capteurs capacitifs est leur utilisation comme interface homme machine sur les claviers tactiles des « *Smartphones* ».

Le problème technique consiste ici à utiliser un capteur capacitif introduit dans un sol (chape ou ragréage ou colle ou revêtements de sol) afin d'effectuer de la détection de présence et/ou de chute, puis d'effectuer une localisation pour ensuite suivre l'activité.

Comme expliqué précédemment, dans le document WO 2014/053719, localiser la position spatiale et la surface occupée au sol par un individu dans une pièce n'est pas possible.

Une telle location est rendue possible dans le cadre de la présente invention en exploitant deux directions de l'espace.

Dans l'exemple décrit ici, et comme illustré en figures 1 et 2, le capteur 100 présente ainsi une première C1 et une deuxième C2 couches.

Plus particulièrement, dans cet exemple, la première couche C1 comprend une pluralité d'électrodes E₁ᵢ, dites premières électrodes ; la variable i est un entier positif compris entre 1 et N.

Dans l'exemple décrit ici et illustré en figure 1, N est égal à 9. Bien évidemment, il s'agit d'un exemple parmi d'autres.

De la même façon, la deuxième couche C2 comprend une pluralité d'électrodes E₂ⱼ, dites deuxièmes électrodes ; la variable j est un entier positif compris entre 1 et M.

Dans l'exemple décrit ici et illustré en figure 1, M est égal à 9. Bien évidemment, il s'agit d'un exemple parmi d'autres.

Ici, N est égal à M. Ceci n'est nullement limitatif. Il est tout à fait possible d'avoir des valeurs différentes pour ces variables ; ceci dépend principalement des dimensions du sol qu'il faut instrumenter.

Dans cet exemple, les électrodes E₁ᵢ de la première couche C1 s'étendent toutes dans une même direction dl, dite première direction ; les électrodes E₂ⱼ de la deuxième couche C2 s'étendent quant à elles dans une autre direction d2, dite deuxième direction.

Dans l'exemple décrit, les directions d1 et d2 sont perpendiculaires entre elles.

Ceci correspond à un mode de réalisation préféré permettant d'obtenir la meilleure précision dans la localisation. Il est toutefois envisageable d'avoir des directions qui ne sont pas perpendiculaires entre elles.

A tout le moins, les directions d1 et d2 ne soient pas identiques entre elles (en d'autres termes, il faut qu'elles ne soient ni parallèles ni confondues entre elles) ; le capteur 100 fonctionne correctement lorsque les directions d1 et d2 sont sécantes entre elles.

Dans l'exemple décrit ici et comme illustré en figure 2, les première C1 et deuxième C2 couches s'étendent chacune selon des plans respectifs P1 et P2.

Bien que non-obligatoire, il est préférable que ces plans P1 et P2 soient parallèles entre eux.

Dans l'exemple décrit ici, il est prévu que les première C1 et deuxième C2 couches soient superposées l'une contre l'autre. Ceci est souhaitable pour faciliter la pose des couches C1 et C2.

Selon l'invention, les couches C1 et C2 sont isolées électriquement l'une par rapport à l'autre.

Pour ce faire, il est prévu d'isoler chaque électrode E₁ᵢ et E₂ⱼ dans une gaine protectrice G1 et G2, de préférence composée au moins partiellement dans un matériau polymérique.

Dans l'exemple décrit ici, les gaines G1, G2 entourant les électrodes de chaque couche respectivement C1 et C2 sont maintenues entre-elles par un support respectivement S1 et S2 de sorte que les électrodes de chaque couche C1 et C2 restent sensiblement parallèles entre elles.

Dans l'exemple décrit ici, chaque support S1 et S2 présente une structure aérée ou présentant des perforations.

Les supports S1 et S2 respectivement des couches C1 et C2 peuvent être constitués d'un filet à maille centimétrique et être auto-adhésifs.

Dans l'exemple décrit ici, un filet de fibre de verre est utilisé pour former chaque support S1 et S2 ; ceci permet une bonne intégration de chaque couche C1 et C2 dans le sol.

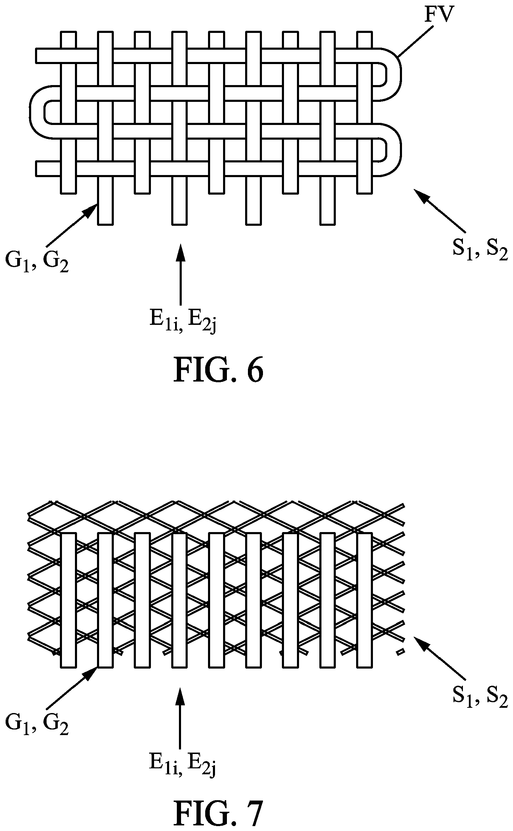

Dans un exemple de réalisation particulier illustré en figure 6, les électrodes de chaque couche sont assemblées entre elles par tissage, par exemple un tissage du type fil de trame et fils de chaîne.

Dans cet exemple, les gaines G1 et G2 entourant les électrodes de chaque couche C1 et C2 forment les fils dit fils de chaîne, et la fibre de verre du filet forme le fil de trame.

Dans l'exemple de réalisation illustré en figure 7, les gaines G1 et G2 entourant les électrodes de chaque couche C1 et C2 sont collées directement au support, par exemple un filet à maille centimétrique (ou grillage).

Le capteur 100 selon l'exemple de réalisation décrit ici et illustré en figures 1 et 2 est particulièrement performant pour établir une localisation spatiale d'un objet et/ou d'un individu ainsi qu'une estimation de la surface qu'il utilise au sol.

En effet, chaque électrode E₁ᵢ et E₂ⱼ du capteur 100 est connectée à une électronique située à l'extérieur du sol.

Cette électronique est composée notamment d'un module électronique de pilotage 10 lui-même connecté avec un module informatique de traitement 20.

Cette électronique permet le pilotage synchrone des électrodes et l'acquisition des mesures des charges électriques de chaque électrode.

Plus particulièrement, dans cet exemple, le module 10 est composé d'une pluralité de cartes électroniques CE1, CE1', CE1", CE2, CE2', et CE2".

Ici, chaque carte CE1, CE1', CE1", CE2, CE2', et CE2" est reliée à un jeu de trois électrodes d'une même couche C1 ou C2.

Dans cet exemple, et comme illustré ici en figure 1, la carte CE1 est donc connectée aux électrodes E₁₁, E₁₂, et E₁₃ de la première couche C1, la carte CE1' est connectée aux électrodes E₁₄, E₁₅, et E₁₆ de la première couche C1, et ainsi de suite.

De préférence, les connexions entre les électrodes E₁ᵢ et E₂ⱼ et les cartes électroniques de mesure CE1, CE1', CE1", CE2, CE2', et CE2" se font à l'extérieur (c'est-à-dire à l'extérieur du sol), de manière à pouvoir réparer aisément une connexion défectueuse.

Une même carte électronique peut donc être utilisée pour piloter et mesurer de façon indépendante la capacité d'une ou plusieurs électrodes E₁ᵢ et E₂ⱼ.

Dans l'exemple décrit ici, les cartes CE1, CE1', CE1", CE2, CE2', et CE2" sont reliées à un réseau, ce qui leur permet de communiquer entre elles pour avoir un pilotage synchrone.

Cette interconnexion leur permet également de communiquer avec un module informatique de traitement 20, qui est distant de la salle instrumentée.

Ce module informatique de traitement 20 contient un programme qui analyse et interprète les données de mesure transmises par chacune des cartes CE1, CE1', CE1", CE2, CE2', et CE2" du module électronique de pilotage 10.

Plusieurs approches sont possibles pour permettre de réaliser la localisation.

Selon l'état de l'art, le module 10 est configuré pour mesurer les charges dépendant des couplages à la terre de chaque électrode E₁ᵢ et E₂ⱼ.

Dans cette approche, le module électronique de pilotage 10 est ainsi configuré pour que les cartes CE1, CE1', CE1", CE2, CE2', et CE2" appliquent simultanément à toutes les électrodes E₁ᵢ et E₂ⱼ une même tension V de manière à mesurer principalement le couplage à la terre de chaque électrode E₁ᵢ et E₂ⱼ.

La présence d'un individu à proximité d'une électrode fait varier le couplage à la terre de cette électrode.

Il est alors possible de localiser un individu dans la pièce, en fonction de la position des électrodes dont les charges varient et d'estimer la surface au sol dudit individu en fonction du nombre d'électrodes.

Le capteur 100 comporte donc à cet effet un module informatique de traitement 20 qui est relié à chacune des cartes CE1, CE1', CE1", CE2, CE2', et CE2" du module électronique de pilotage 10 pour recueillir et traiter les informations relatives aux variations de capacité de chacune des électrodes.

Plus particulièrement, pour estimer la position de l'individu dans la pièce et la surface qu'il occupe au sol, le module de traitement 20 identifie les intersections entre les lignes et les colonnes dont la capacité propre a dépassé un seuil prédéfini.

Ce traitement permet d'estimer une position spatiale et la surface au sol de l'individu.

Plusieurs applications peuvent être envisagées. Par exemple, une personne allongée sur le sol modifie la capacité d'un grand nombre d'électrodes.

Le capteur 100 peut alors être utilisé pour surveiller des personnes âgées et transmettre une alerte si une chute est détectée.

Il est observé que, selon cette approche, lorsque plusieurs « intersections » entre les électrodes sont touchées en même temps, la précision pour localiser les points touchés est limitée.

En effet, les électrodes E₁ᵢ et E₂ⱼ du capteur 100 forment un quadrillage avec des lignes pour les électrodes E₁ᵢ de la première couche C1 et des colonnes pour les électrodes E₂ⱼ de la deuxième couche C2.

Ainsi, dans cet exemple, le capteur 100 ne dispose uniquement que des informations sur les lignes et les colonnes qui ont été touchées.

Les intersections qui n'ont en fait pas été touchées vont malgré tout être considérées comme touchées.

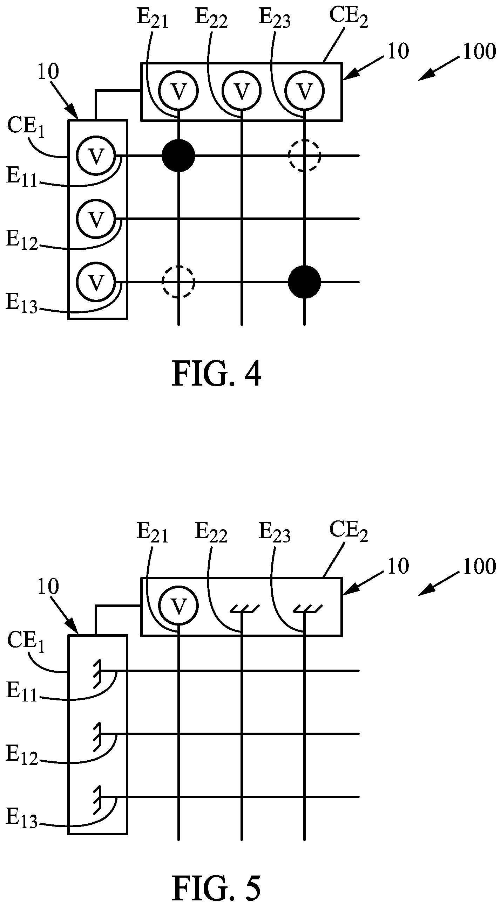

Par exemple, comme illustré en figure 4, lorsqu'un individu est effectivement à proximité de deux intersections différentes (points noircis sur la figure 4), le capteur 100 va spontanément considérer ici que quatre intersections ont été touchées ce qui introduit donc deux fausses informations (ici les deux points en pointillé sur la figure 4).

Pour réaliser ensuite un suivi de l'activité de l'individu et limiter les temps de traitement, le balayage prend en compte uniquement les couplages à la terre qui se situent à proximité de la première position estimée (dans un rayon d'un mètre par exemple).

Ceci permet de suivre l'activité de la personne ; en effet, ce pas d'un mètre correspond sensiblement à la longueur d'un pas d'un individu.

La Demanderesse soumet par ailleurs que le phénomène de détection de « fausses touches » inhérent aux mesures des capacités propres peut être considéré comme négligeable qui est faite ici à la détection et la localisation de chute.

En effet, la position d'un individu debout peut être estimée avec une précision satisfaisante pour pouvoir suivre son déplacement.

Cette approche est donc pertinente : la localisation est suffisamment précise et le temps de calcul très court. Cette approche peut être privilégiée par exemple dans des pièces où le nombre de personnes est faible, par exemple une chambre individuelle d'une maison de retraite.

Pour remédier aux phénomènes de détection de « fausses touches » énoncés ci-dessus, une nouvelle approche être proposée dans le cadre de la présente invention.

Cette approche repose sur la mesure des couplages entre les électrodes indépendamment du couplage à la terre.

Les couplages entre les électrodes d'un capteur correspondent aux capacités à chaque intersection entre les électrodes E₁ᵢ et E₂ⱼ de la première C1 et de la deuxième couche C2.

Pour mesurer une capacité à l'intersection de deux électrodes, le module de pilotage 10 est alors configuré pour que les cartes électroniques appliquent un potentiel électrique à une seule électrode et que les autres électrodes soient toutes mises à la masse.

Ceci est représenté en figure 5.

Ainsi, lorsqu'un individu se trouve à proximité de l'intersection de deux électrodes (une à la masse et une sur laquelle un potentiel est appliqué), la variation de la capacité est obtenue en mesurant les charges sur l'électrode mise à la masse.

Lorsqu'un capteur 100 présente N électrodes dans chaque direction, un balayage complet des capacités aux intersections nécessite alors de faire N mesures, en appliquant une tension à chaque fois à une seule électrode et en mettant tous les autres capteurs à la masse.

Lorsque la première colonne (c'est-à-dire ici l'électrode E₂₁ de la deuxième couche C2) est la seule à laquelle est appliquée une tension (comme illustré en figure 5), la mesure sur toutes les lignes (c'est-à-dire ici toutes les électrodes E₁ᵢ de la première couche C1) permet d'obtenir l'ensemble des inter-capacités de la première colonne avec chaque ligne.

Cette opération doit ensuite être répétée N fois en changeant à chaque fois l'électrode polarisée.

Selon l'approche développée ici, il ne s'agit pas de mesurer l'ensemble des inter-capacités du capteur 100, mais de mesurer les inter-capacités pour lesquels une variation de couplage à la terre a été détectée.

Ainsi, selon cette approche, on mesure d'abord le couplage à la terre de chacune des électrodes du capteur.

Le module de traitement 20 réalise ensuite un traitement informatique de ces couplages à la terre pour sélectionner ceux qui sont pertinents : le module de traitement 20 est ainsi configuré de telle manière que, lorsqu'une ou plusieurs lignes ou colonnes présentent un couplage à la terre dont la valeur dépasse un premier seuil prédéfini, alors, dans ce cas, le module de traitement 20 indique au module de pilotage 10 de mesurer les inter-capacités aux croisements de chaque ligne et chaque colonne correspondantes.

Ainsi, selon cette approche, toutes les inter-capacités ne sont pas mesurées.

Suite à ces nouvelles mesures, les inter-capacités dont la valeur dépasse un seuil déterminé sont alors considérées comme étant « activées », c'est-à-dire qu'un individu (ou un objet) est considéré comme présent au-dessus des croisements correspondants aux deux électrodes.

Selon cette approche, le traitement se poursuit ensuite en mesurant uniquement les inter-capacités à proximité de l'endroit où l'individu est localisé.

Dans un mode de réalisation préférentiel, le traitement se poursuit uniquement pour les inter-capacités dans un rayon d'un mètre autour de la localisation estimée, ce qui correspond à la longueur approximative d'un pas humain.

Il est ainsi possible de suivre avec une grande précision l'activité de la personne.

Si par exemple les inter-capacités « activées » restent les mêmes, cela signifie que l'individu reste immobile.

Si les inter-capacités « activées » à proximité changent de localisation, on peut considérer que l'individu s'est déplacé.

Il est également possible de détecter une chute sur le sol dans le cas où un grand nombre de croisements à proximité s'active simultanément.

On notera ici que cette nouvelle approche permet une localisation précise, notamment lorsque plusieurs inter-capacités varient en même temps. Il est alors possible selon cette approche de différencier plusieurs personnes marchant dans une même pièce.

Quelle que soit l'approche retenue, le module de traitement 20 est configuré pour intégrer l'ensemble des instructions de code permettant de gérer et de traiter ces informations de manière à réaliser la localisation.

Ce module de traitement peut ensuite coopérer avec un module d'affichage 30 pour générer sur l'écran de contrôle 31 une imagerie représentative de l'activité de l'individu dans la pièce.

On notera également que, de par sa structure, le capteur 100 selon l'invention peut être intégré aisément dans un sol.

Différentes structures de sol 200 sont ainsi envisagées dans le cadre de la présente invention, chacune de ces structures comportant un capteur 100 tel que décrit ci-dessus.

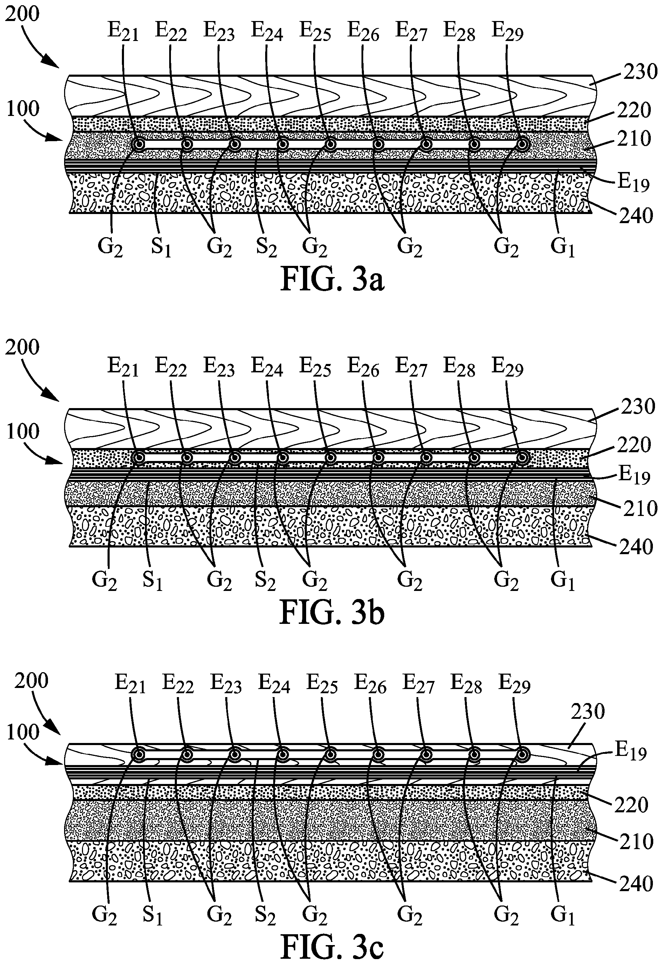

Ainsi, l'exemple de réalisation de la figure 3a prévoit l'incorporation d'un capteur 100 dans une couche de ragréage 210. Dans cet exemple, afin d'assurer une mise en oeuvre facile pour le poseur, chacune des électrodes des première C1 et deuxième C2 couches du capteur 100 est noyée dans une gaine G1 et G2 pour assurer l'isolation électrique de chaque électrode.

Dans ce mode de réalisation, l'instrumentation du sol peut par exemple s'effectuer de la façon suivante :
- une pose de la chape 240,
- une application d'une couche de primaire d'accrochage sur la chape 240,
- une première étape de pose au cours de laquelle on positionne le support S1 contenant la première couche C1 de sorte que les premières électrodes E₁ᵢ de cette couche C1 s'étendent dans une première direction dl déterminée,
- une deuxième étape de pose au cours de laquelle on positionne le support S2 de la deuxième couche C2 de sorte que les deuxièmes électrodes E₂ⱼ de cette couche C2 s'étendent dans une deuxième direction d2 qui est perpendiculaire à la première direction dl.
- un coulage d'une couche de ragréage 210 sur les couches C1 et C2 du capteur 100,
- une application d'une couche de colle 220 et d'une couche de revêtement 230 sur le ragréage 210 sec instrumenté avec les capteurs 100.

Ainsi, un sol instrumenté par le capteur capacitif 100 selon l'invention permet de détecter et de localiser efficacement et avec précision les personnes près du sol.

L'exemple de réalisation de la figure 3b prévoit une instrumentation du sol avec un capteur 100 posé directement ou indirectement sur la couche de ragréage 210.

Dans cet exemple, l'instrumentation du sol est effectuée de la façon suivante : les supports S1 et S2 des couches C1 et C2 du capteur 100 sont posés l'un après l'autre sur le ragréage 210. Une couche de colle 220 est ensuite appliquée sur les supports S1 et S2, puis une couche de revêtement 230 est appliquée sur cette couche de colle 220.

Alternativement, les supports S1 et S2 peuvent être fixées sur le revêtement 230 par une couche de colle 220, puis on pose sur la couche de ragréage le revêtement 230 sur lequel sont fixés les supports S1 et S2

Selon un autre exemple de mise en oeuvre illustré en figure 3c, le capteur 100 est directement introduit dans la couche de revêtement 230.

Dans cet exemple, on reprend les précédentes étapes de l'installation, en ayant préalablement fabriqué une couche de revêtement 230 instrumentée (par exemple par tissage) le capteur 100 avec la couche de revêtement 230.

Ainsi, la structure du capteur telle que prévue dans la cadre de la présente invention, avec deux couches d'électrodes isolées électriquement les unes par rapport aux autres, est particulièrement avantageuse et permet la conception d'un capteur capacitif 100 garantissant une localisation fine et précise des individus et/ou des objets qui sont situés à proximité dudit capteur.

Le capteur 100 obtenu selon la présente invention peut aisément servir à instrumenter un sol pour une application par exemple dans des bâtiments médicalisés et/ou des maisons de retraite.

Il devra être observé que cette description détaillée porte sur des exemples de réalisation particuliers de la présente invention, mais qu'en aucun cas cette description ne revêt un quelconque caractère limitatif à l'objet de l'invention ; bien au contraire, elle a pour objectif d'ôter toute éventuelle imprécision ou toute mauvaise interprétation des revendications qui suivent.

## Revendications

1. Capteur capacitif (100) pour détecter, localiser la présence, évaluer la surface au sol et/ou suivre l'activité d'un individu et/ou d'un objet,
ledit capteur (100) comportant :
- une première couche (C1) comprenant au moins une première électrode (E₁ᵢ, i∈[1,N]) s'étendant dans une première direction (d1);
- une deuxième couche (C2) comprenant au moins une deuxième électrode (E₂ⱼ, j∈[1,M]) s'étendant dans une deuxième direction (d2);
dans lequel la première direction (d1) est différente de la deuxième direction (d2), et dans lequel la première couche (C1) est électriquement isolée de la deuxième couche (C2),
ledit capteur (100) comportant en outre :
- un module électronique de pilotage (10) configuré :
• pour appliquer de manière indépendante une tension sur chacune des électrodes (E₁ᵢ, E₂ⱼ) dont au moins une tension nulle sur au moins l'une desdites électrodes (E₁ᵢ, E₂ⱼ), et
• pour mesurer les charges électriques accumulées sur chaque électrode (E₁ᵢ, E₂ⱼ), caractérisé en ce ledit capteur (100) comporte en outre:
- un module informatique de traitement (20) configuré pour analyser la distribution spatiale des variations des charges mesurées pour chacune desdites électrodes (E1i, E2j) en déterminant :
• une information dépendante du couplage à la terre d'une première électrode de mesure parmi la au moins une première électrode (E₁ᵢ) si la tension appliquée sur la première électrode de mesure n'est pas nulle, ladite première électrode de mesure étant comprise dans la première couche,
et, suivant l'information dépendante du couplage à la terre déterminée, en déterminant :
• une information indépendante du couplage à la terre d'une deuxième électrode de parmi la au moins une deuxième électrode (E₂ⱼ) si la tension appliquée sur la deuxième électrode de mesure est nulle et que la tension appliquée sur la première électrode de mesure n'est pas nulle, ladite deuxième électrode de mesure étant comprise dans la deuxième couche,
de manière à détecter, localiser la présence, évaluer la surface au sol et/ou suivre l'activité d'un individu et/ou d'un objet.

2. Capteur (100) selon la revendication 1, dans lequel les première (d1) et deuxième (d2) directions sont sensiblement perpendiculaires entre elles.

3. Capteur (100) selon la revendication 1 ou 2, dans lequel lesdites première (C1) et deuxième (C2) couches s'étendent dans des plans (P1 ; P2) sensiblement parallèles entre eux.

4. Capteur (100) selon l'une quelconque des revendications précédentes, comprenant un module d'affichage (30) comprenant un écran de contrôle (31) et coopérant avec le module informatique de traitement (20) pour générer sur l'écran (31) une imagerie représentative de la position spatiale de l'objet et/ou de l'individu en fonction de la distribution spatiale des charges mesurées pour chacune desdites électrodes (E₁ᵢ, E₂ⱼ).

5. Capteur (100) selon l'une quelconque des revendications 1 à 4, dans lequel chaque électrode (E₁ᵢ, E₂ⱼ) des première (C1) et deuxième (C2) couches est isolée électriquement par une gaine protectrice (G1, G2) constituée au moins partiellement dans un matériau polymérique.

6. Capteur (100) selon la revendication 5, dans lequel chaque gaine protectrice (G1, G2) est maintenue par un support (S1, S2) présentant une structure perforée ou aérée.

7. Capteur (100) selon la revendication 6, dans lequel chaque support (S1, S2) présente au moins une face auto-adhésive.

8. Capteur (100) selon la revendication 6 ou 7, dans lequel chaque support (S1, S2) comprend un filet à maille centimétrique constitué au moins partiellement dans un matériau sélectionné parmi les matériaux suivants : fibre de verre, polyester, polypropylène, polyéthylène, ou encore polyamide.

9. Capteur (100) selon la revendication 8, dans lequel chaque électrode (E₁ᵢ, E₂ⱼ) des première (C1) et deuxième (C2) couches est isolée électriquement par une gaine protectrice (G1, G2), et dans lequel, pour chaque couche (C1, C2), le filet à maille et les électrodes sont assemblées entre elles par un tissage du type fils de chaîne et fil de trame, lesdits fils de chaîne et ledit fil de trame étant formés respectivement par les gaines (G1, G2) de chaque électrode et le filet à maille.

10. Structure de sol (200) pour localiser un objet et/ou un individu comprenant un capteur capacitif (100) selon l'une quelconque des revendications 1 à 9.

11. Structure de sol (200) selon la revendication 10 présentant une couche de chape (240) et une couche de ragréage (210), ledit capteur (100) étant fixé par collage sur au moins une portion de la face supérieure de la couche de chape (240), ledit capteur (100) étant intégré dans la couche de ragréage (210) par coulage de ladite couche de ragréage (210) sur la couche de chape (240).

12. Procédé de fabrication d'une structure de sol (200) selon la revendication 10 ou 11 comprenant :
• une première étape au cours de laquelle une première couche (C1) comprenant au moins une première électrode (E₁ᵢ) est positionnée de sorte que ladite au moins première électrode (E₁ᵢ) s'étende dans une première direction déterminée (d1), et
• une deuxième étape au cours de laquelle une deuxième couche (C2) comprenant au moins une deuxième électrode (E₂ⱼ) est positionnée de sorte que ladite deuxième couche (C2) soit électriquement isolée de la première couche (C1) et que ladite au moins une deuxième électrode (E₂ⱼ) s'étende dans une deuxième direction (d2) différente de la première direction (d1).

13. Procédé selon la revendication 12, dans lequel, lors de la deuxième étape, la deuxième couche (C2) est positionnée de sorte que la deuxième direction (d2) dans laquelle s'étend ladite au moins une deuxième électrode (E₂ⱼ) soit sensiblement perpendiculaire à la première direction (d1) dans laquelle s'étend ladite au moins une première électrode (E₁ᵢ, E₂ⱼ).

## Patentansprüche

1. Kapazitiver Sensor (100) zum Detektieren, Lokalisieren der Anwesenheit, Abschätzen der Bodenfläche und/oder Verfolgen der Aktivität eines Individuums und/oder eines Objekts,
wobei der Sensor (100) aufweist:
- eine erste Schicht (C1), die wenigstens eine erste Elektrode (E₁ᵢ, i∈ [1,N]) umfasst, die sich in einer ersten Richtung (d1) erstreckt;
- eine zweite Schicht (C2), die wenigstens eine zweite Elektrode (E₂ⱼ, j∈ [1,M]) umfasst, die sich in einer zweiten Richtung (d2) erstreckt;
wobei die erste Richtung (d1) von der zweiten Richtung (d2) verschieden ist und wobei die erste Schicht (C1) von der zweiten Schicht (C2) elektrisch isoliert ist,
wobei der Sensor (100) außerdem aufweist:
- ein elektronisches Steuermodul (10), das dafür ausgelegt ist:
• auf unabhängige Weise eine Spannung an jede der Elektroden (E₁ᵢ, E₂ⱼ) anzulegen, darunter wenigstens eine Nullspannung an wenigstens eine der Elektroden (E₁ᵢ, E₂ⱼ), und
• die an jeder Elektrode (E₁ᵢ, E₂ⱼ) akkumulierten elektrischen Ladungen zu messen,
**dadurch gekennzeichnet, dass** der Sensor (100) außerdem aufweist:
- ein Informationsverarbeitungsmodul (20), das dafür ausgelegt ist, die räumliche Verteilung der Änderungen der gemessenen Ladungen für jede der Elektroden (E₁ᵢ, E₂ⱼ) zu analysieren, indem es bestimmt:
• eine Information, die von der Massekopplung einer ersten Messelektrode von der wenigstens einen ersten Elektrode (E₁ᵢ) abhängig ist, falls die an die erste Messelektrode angelegte Spannung nicht null ist, wobei die erste Messelektrode in der ersten Schicht enthalten ist,
und indem es, je nach der bestimmten Information, die von der Massekopplung abhängig ist, bestimmt:
• eine Information, die von der Massekopplung einer zweiten Elektrode von der wenigstens einen zweiten Elektrode (E₂ⱼ) unabhängig ist, falls die an die zweite Messelektrode angelegte Spannung null ist und die an die erste Messelektrode angelegte Spannung nicht null ist, wobei die zweite Messelektrode in der zweiten Schicht enthalten ist,
um so ein Individuum und/oder ein Objekt zu detektieren, seine Anwesenheit zu lokalisieren, seine Bodenfläche abzuschätzen und/oder seine Aktivität zu verfolgen.

2. Sensor (100) nach Anspruch 1, wobei die erste (d1) und zweite (d2) Richtung im Wesentlichen zueinander senkrecht sind.

3. Sensor (100) nach Anspruch 1 oder 2, wobei sich die erste (C1) und zweite (C2) Schicht in Ebenen (P1; P2) erstrecken, die im Wesentlichen zueinander parallel sind.

4. Sensor (100) nach einem der vorhergehenden Ansprüche, welcher ein Anzeigemodul (30) umfasst, das einen Kontrollbildschirm (31) umfasst und mit dem Informationsverarbeitungsmodul (20) zusammenwirkt, um in Abhängigkeit von der räumlichen Verteilung der gemessenen Ladungen für jede der Elektroden (E₁ᵢ, E₂ⱼ) auf dem Bildschirm (31) eine Bildgebung zu erzeugen, die für die räumliche Position des Objekts und/oder des Individuums repräsentativ ist.

5. Sensor (100) nach einem der Ansprüche 1 bis 4, wobei jede Elektrode (E₁ᵢ, E₂ⱼ) der ersten (C1) und zweiten (C2) Schicht durch eine Schutzhülle (G1, G2) elektrisch isoliert ist, die wenigstens teilweise aus einem Polymermaterial besteht.

6. Sensor (100) nach Anspruch 5, wobei jede Schutzhülle (G1, G2) von einem Träger (S1, S2) gehalten wird, der eine perforierte oder aufgelockerte Struktur aufweist.

7. Sensor (100) nach Anspruch 6, wobei jeder Träger (S1, S2) wenigstens eine selbstklebende Seite aufweist.

8. Sensor (100) nach Anspruch 6 oder 7, wobei jeder Träger (S1, S2) ein Zentimeter-Maschennetz umfasst, das wenigstens teilweise aus einem Material besteht, das aus den folgenden Materialien ausgewählt ist: Glasfasern, Polyester, Polypropylen, Polyethylen oder auch Polyamid.

9. Sensor (100) nach Anspruch 8, wobei jede Elektrode (E₁ᵢ, E₂ⱼ) der ersten (C1) und zweiten (C2) Schicht durch eine Schutzhülle (G1, G2) elektrisch isoliert ist, und wobei für jede Schicht (C1, C2) das Maschennetz und die Elektroden durch ein Gewebe des Typs Kettfäden und Schussfaden miteinander verbunden sind, wobei die Kettfäden und der Schussfaden von den Hüllen (G1, G2) jeder Elektrode bzw. dem Maschennetz gebildet sind.

10. Bodenstruktur (200) zum Lokalisieren eines Objekts und/oder eines Individuums, welche einen kapazitiven Sensor (100) nach einem der Ansprüche 1 bis 9 umfasst.

11. Bodenstruktur (200) nach Anspruch 10, welche eine Estrichschicht (240) und eine Ausgleichsmasseschicht (210) aufweist, wobei der Sensor (100) durch Kleben auf wenigstens einem Abschnitt der Oberseite der Estrichschicht (240) befestigt ist, wobei der Sensor (100) in die Ausgleichsmasseschicht (210) durch Gießen der Ausgleichsmasseschicht (210) auf die Estrichschicht (240) integriert ist.

12. Verfahren zur Herstellung einer Bodenstruktur (200) nach Anspruch 10 oder 11, welches umfasst:
• einen ersten Schritt, in welchem eine erste Schicht (C1), die wenigstens eine erste Elektrode (E₁ᵢ) umfasst, derart positioniert wird, dass sich die wenigstens eine erste Elektrode (E₁ᵢ) in einer bestimmten ersten Richtung (d1) erstreckt, und
• einen zweiten Schritt, in welchem eine zweite Schicht (C2), die wenigstens eine zweite Elektrode (E₂ⱼ) umfasst, derart positioniert wird, dass die zweite Schicht (C2) von der ersten Schicht (C1) elektrisch isoliert ist, und dass sich die wenigstens eine zweite Elektrode (E₂ⱼ) in einer zweiten Richtung (d2) erstreckt, die von der ersten Richtung (d1) verschieden ist.

13. Verfahren nach Anspruch 12, wobei im zweiten Schritt die zweite Schicht (C2) derart positioniert wird, dass die zweite Richtung (d2), in welcher sich die wenigstens eine zweite Elektrode (E₂ⱼ) erstreckt, im Wesentlichen senkrecht zu der ersten Richtung (d1) ist, in welcher sich die wenigstens eine erste Elektrode (E₁ᵢ, E₂ⱼ) erstreckt.

## Claims

1. Capacitive sensor (100) for detecting, locating the presence of, assessing the floor area of and/or tracking the activity of a person and/or of an object,
said sensor (100) including:
- a first layer (C1) comprising at least one first electrode (E₁ᵢ, i∈[1,N]) running in a first direction (d1);
- a second layer (C2) comprising at least one second electrode (E₂ⱼ, j∈[1,M]) running in a second direction (d2);
wherein the first direction (d1) is different from the second direction (d2), and wherein the first layer (C1) is electrically insulated from the second layer (C2),
said sensor (100) further including:
- an electronic driver module (10) configured:
• to independently apply a voltage to each of the electrodes (E₁ᵢ, E₂ⱼ), including at least one zero voltage to at least one of said electrodes (E₁ᵢ, E₂ⱼ); and
• to measure the electric charges that have built up on each electrode (E₁ᵢ, E₂ⱼ), **characterized in that** said sensor (100) further includes:
- a computer processing module (20) configured to analyse the spatial distribution of the variations in the charges measured for each of said electrodes (E₁ᵢ, E₂ⱼ) by determining:
• information that is dependent on the coupling to ground of a first measurement electrode from among the at least one first electrode (E₁ᵢ) if the voltage applied to the first measurement electrode is nonzero, said first measurement electrode being within the first layer;
and, according to the determined information that is dependent on the coupling to ground, by determining:
• information that is independent of the coupling to ground of a second electrode from among the at least one second electrode (E₂ⱼ) if the voltage applied to the second measurement electrode is zero and the voltage applied to the first measurement electrode is nonzero, said second measurement electrode being within the second layer,
so as to detect, to locate the presence of, to assess the floor area of and/or to track the activity of a person and/or of an object.

2. Sensor (100) according to Claim 1, wherein the first (d1) and second (d2) directions are substantially perpendicular to one another.

3. Sensor (100) according to Claim 1 or 2, wherein said first (C1) and second (C2) layers lie in planes (P1; P2) that are substantially parallel to one another.

4. Sensor (100) according to any one of the preceding claims, comprising a display module (30) comprising a control screen (31) and cooperating with the computer processing module (20) to generate, on the screen (31), an image that is representative of the spatial position of the object and/or of the person according to the spatial distribution of the charges measured for each of said electrodes (E₁ᵢ, E₂ⱼ).

5. Sensor (100) according to any one of Claims 1 to 4, wherein each electrode (E₁ᵢ, E₂ⱼ) of the first (C1) and second (C2) layers is electrically insulated by a protective sheath (G1, G2) consisting at least partially of a polymer material.

6. Sensor (100) according to Claim 5, wherein each protective sheath (G1, G2) is held by a support (S1, S2) having a perforated or aerated structure.

7. Sensor (100) according to Claim 6, wherein each support (S1, S2) has at least one self-adhesive face.

8. Sensor (100) according to Claim 6 or 7, wherein each support (S1, S2) comprises a centimetre-scale-mesh net consisting at least partially of a material selected from among the following materials: glass fibre, polyester, polypropylene, polyethylene, or polyamide.

9. Sensor (100) according to Claim 8, wherein each electrode (E₁ᵢ, E₂ⱼ) of the first (C1) and second (C2) layers is electrically insulated by a protective sheath (G1, G2), and wherein, for each layer (C1, C2), the mesh net and the electrodes are joined together by a weave with warp threads and a weft thread, said warp threads and said weft thread being formed by the sheaths (G1, G2) of each electrode and the mesh net, respectively.

10. Floor structure (200) for locating an object and/or a person comprising a capacitive sensor (100) according to any one of Claims 1 to 9.

11. Floor structure (200) according to Claim 10, having a screed layer (240) and a levelling layer (210), said sensor (100) being attached, by bonding, to at least a portion of the upper face of the screed layer (240), said sensor (100) being incorporated into the levelling layer (210) by said levelling layer (210) being poured over the screed layer (240).

12. Method for producing a floor structure (200) according to Claim 10 or 11, comprising:
• a first step in which a first layer (C1) comprising at least one first electrode (E₁ᵢ) is positioned such that said at least one first electrode (E₁ᵢ) runs in a first determined direction (d1); and
• a second step in which a second layer (C2) comprising at least one second electrode (E₂ⱼ) is positioned such that said second layer (C2) is electrically insulated from the first layer (C1) and such that said at least one second electrode (E₂ⱼ) runs in a second direction (d2) that is different from the first direction (d1).

13. Method according to Claim 12, wherein, in the second step, the second layer (C2) is positioned such that the second direction (d2) in which said at least one second electrode (E₂ⱼ) runs is substantially perpendicular to the first direction (d1) in which said at least one first electrode (E₁ᵢ, E₂ⱼ) runs.
